# EUROPEAN PATENT APPLICATION

(11) **EP 1 679 379 A1**
(43) Date of publication of application: **12.07.2006**
(21) Application number: 05075017.3
(22) Date of filing: 06.01.2005
(51) Int. Cl.: C12Q 1/68

(54) **Diagnosis of metastases in HNSCC tumours**

(71) Applicant: UMC Utrecht Holding B.V., 3584 CK Utrecht (NL)
(72) Inventor: Holstege, Frank Charles Patrick, 3991 KC Houten (NL); Slootweg, Pieter Johannes, 3723 CL Bilthoven (NL); Roepman, Paul, 6702 AH Wageningen (NL)
(74) Representative: Winckels, Johannes Hubertus F.

(57) **Abstract**

The invention relates to the detection or prediction of metastases of head and neck squamous cell carcinoma (HNSCC) with the use of gene expression profiles. A gene signature has been identified which is able to detect or predict the occurrence of these metastases better than current clinical methods. Part of the invention are micro-arrays comprising this signature and methods for performing the detection and/or prediction.

## Description

### FIELD OF THE INVENTION

The invention relates to the field of tumour diagnosis, in particular to predict the existence of metastases of a tumour, more in particular to the detection of lymph node metastases of head and neck squamous cell carcinoma (HNSCC) especially those that arise in the oral cavity and oropharynx.

### STATE OF THE PRIOR ART

Head and neck squamous cell carcinoma (HNSCC) consists of a heterogenous group of neoplasms that arise from the epithelium of the upper aero-digestive tract. HNSCC is the fifth most common malignancy in humans and is particularly frequent in regions where alcohol and tobacco use is high (Sankanarayanan, R. *et al.,* (1998) Anticancer Res. 18, 4779-4786). The survival rate of patients with cervical lymph node metastases is reduced by almost 50% (Jones, A.S. *et al*., (1994) Clin. Otolaryngol. 19, 63-69; Hahn, S.S. *et al.,* (1987) J. Radiat. Oncol. Biol. Phys. 13, 1143-1147). As with most forms of cancer, treatment depends largely on progression stage (Forastiere, A. *et al.,* (2001) N. Eng. J. Med. 345, 1890-1900).

Metastasis is the process whereby cancers spread to distinct sites in the body. It is the principal cause of death in individuals suffering from cancer. For some tumor types, the earliest detectable sign of metastasis is the presence of malignant cells in lymph nodes close to the site of the primary tumour. Early detection of local lymph node metastases is currently pivotal for appropriate treatment of many types of cancer. However, because of difficulties in detecting lymph node metastases reliably, many patients currently receive inappropriate treatment.

Most patients with HNSCC, especially those in the oral cavity or oropharynx have the primary tumour removed. Treatment of clinically diagnosed lymph node metastasis positive patients (N+) involves the additional surgical removal of a significant portion of the neck, including all five local lymph node levels: radical neck dissection (RND) (Robbins, K.T. *et al.,* (2002) Arch. Otolaryngol. Head Neck Surg. 128, 751-758). However, after histological examination of the removed tissue, approximately 10-20% of the clinically diagnosed N+ patients appear to be N0 (metastasis free) (Woolgar, J.A., (1999) Br. J. Oral Maxillofac. Surg. 37, 205-209). Clinical diagnosis of N0 lymph node status is even less accurate. Histological examination of electively operated clinically diagnosed N0 patients reveals that about one-third have positive neck nodes (Jones, A.S. *et al.,* (1993) Eur. Arch. Otorhinolaryngol. 250, 446-449). Different strategies exist for neck treatment of N0 diagnosed patients (Pillsbury, H.C., *et al.,* (1997) Laryngoscope 107, 1294-1315). One is the so-called "watch and wait" strategy by which N0 diagnosed patients do not undergo any neck dissection. The involves the risk of fatality by allowing overlooked metastases to develop and spread further. Since the prevalence of false-negative predictions is very high, most clinics perform neck surgery for all diagnosed N0 patients. In this case most often a supra-omohyoidal neck dissection (SOHND) is performed, removing the three upper lymph node levels (Robbins, K.T. *et al., supra*)*.* This treatment is less appropriate than an RND for those N+ patients falsely diagnosed as N0 and, moreover, completely unnecessary for all patients correctly diagnosed as N0. Although SOHND is less rigorous than RND, the treatment cause disfigurement, long-term discomfort, pain and can lead to additional complications such as shoulder disability (e.g. Short, S.O. *et al.,* (1984) Am. J. Surg. 148, 478-482). Both treatments strategies result in over- or undertreatment due to limitations in detecting lymph node metastasis reliably.

DNA microarray based gene expression profiling has previously been shown to be useful for cancer classification (e.g. Valk, P.J. *et al.,* (2004) N. Eng. J. Med. 350, 1605-1616) and prognosis-based treatment (Van't Veer, L.J. *et al.,* (2002) Nature 415, 530-536 and WO 2004/065545). Gene expression profiling has revealed genes that are differentially regulated in metastases (Clark, E.A. *et al.,* (2000) Nature 406, 532-535; Saha, S. *et al.,* (2001) Science 294, 1343-1346) and a 17-gene expression signature was recently found that is common to both primary tumours and metastases (Ramaswamy, S. *et al.,* (2003) Nat. Genet. 33, 49-54). It was also found that a signature derived from fibroblasts which are active in wound healing can be predictive for metastasis in several tumour types (Chang, H.Y., et al. (2004) PLoS Biol. 2(2):e7, to be found at:
http://www.pubmedcentral.nih.gov/articlerender.fcgi?artid=314300).

For HNSCC such expression signatures are starting to be uncovered (Chung, C.H. *et al.,* (2004) Cancer Cell 5, 489-500), but as yet without independent validation for reliability and clinical outcome.

Thus, there is still a large need for a reliable diagnostic tool on basis of expression of genes with which a reliable and accurate prediction can be established for the presence or occurrence of lymph node metastasis in HNSCC.

### SUMMARY OF THE INVENTION

The invention now provides a nucleotide array for the detection of metastasis in head and neck squamous cell cancer (HNSCC) comprising at least 5 of the elements of Table 1, more preferably 70%, more preferably 80%, more preferably 90%, more preferably 95%, more preferably 99% and most preferably all of the elements of Table 1. Preferably such a nucleotide array for the detection of metastasis in HNSCC comprises one or more of the elements of the genes listed in Table 2.
Further provided is a method to establish reference and control gene expression profiles of patients having had metastasis after HNSCC (N+ group) or no metastasis after HNSCC (N0 group) by analysing the gene expression from a tumour biopsy sample of each patient, or from pooled samples of each group of patients, on an array according to the invention.
Another embodiment of the invention is a method to predict the presence or risk of occurrence of lymph node metastasis of a HNSCC patient. comprising:
a. taking a biopsy sample from the tumour of the patient;
b. isolating the nucleic acid from the biopsy sample;
c. analyse the gene expression profile of said nucleic acid by assaying it with a nucleotide array according to the invention;
d. classifying the expression profile as N+ or N0 by determining whether the expression profile would match the expression profile of a group of HNSCC patients known to have developed metastasis.
Preferably, the biopsy samples in the above methods are fresh biopsy samples.
A preferred embodiment for the method to predict the presence or risk of metastasis is a method, wherein the analysis of the gene expression profile comprises:
a. hybridising the nucleic acid form the biopsy sample with the nucleotide array according to the invention;
b. determining the amount of hybridisation of each of the elements of the nucleotide array relative to the amount of hybridisation of each element with a reference sample, said step optionally involving a normalisation step;
c. determining for each element of the array whether the expression of the corresponding gene in the biopsy sample is more or less than the expression of the corresponding gene in the reference sample.
Preferably, the expression profile is classified as N+ (high risk of metastasis) or N0 (low or no risk of metastasis) according to the steps of:
a. determining the collective correlation of the classifier/predictor genes or elements present in the expression profile with the average N+ or N0 profile from primary tumors with previously established N-status; and
b. determining the predictive threshold based on the correlation threshold from primary tumors with previously established N-status
In another preferred embodiment the method is a method, wherein the gene expression profile of a group of HNSCC patients known to have developed metastasis is the expression profile contained in the dataset E-UMCU-11, available in the public microarray database ArrayExpress (http://www.ebi.ac.uk/arraveypress/). Calculation of the correlation as performed in the above methods is preferably done using the cosine correlation method.
Normalization of the expression profile is preferably achieved by correcting the expression data for experimental variations with the help of expression data of a control gene or element which is not affected by the tumour state, preferably by calculating the ratio of the expression data of each gene or element in the array of claim 1 or 2 with the expression of a control gene or element or the mean of a pool of control genes or elements.

### LEGENDS TO THE FIGURES

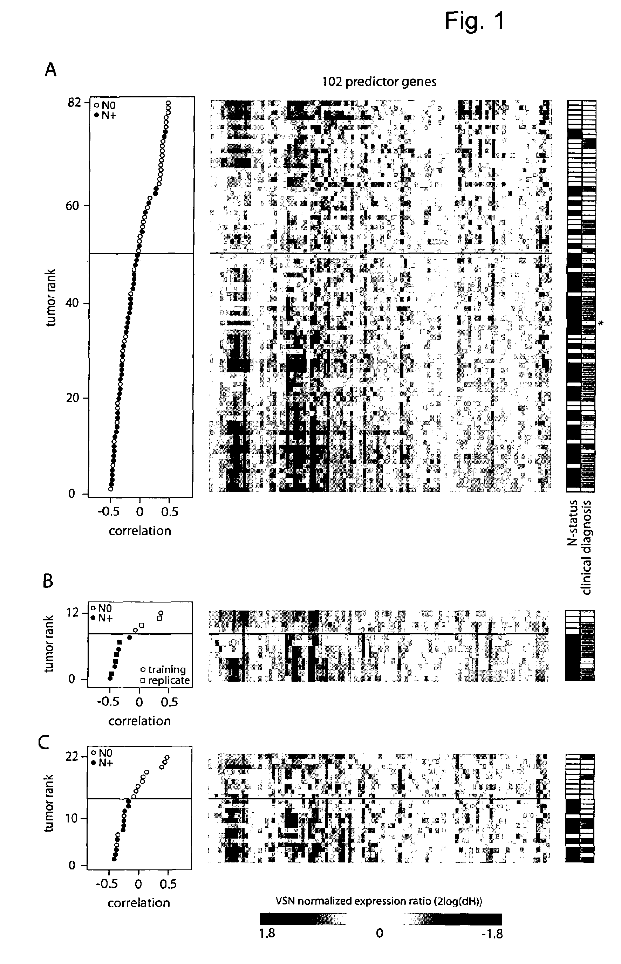

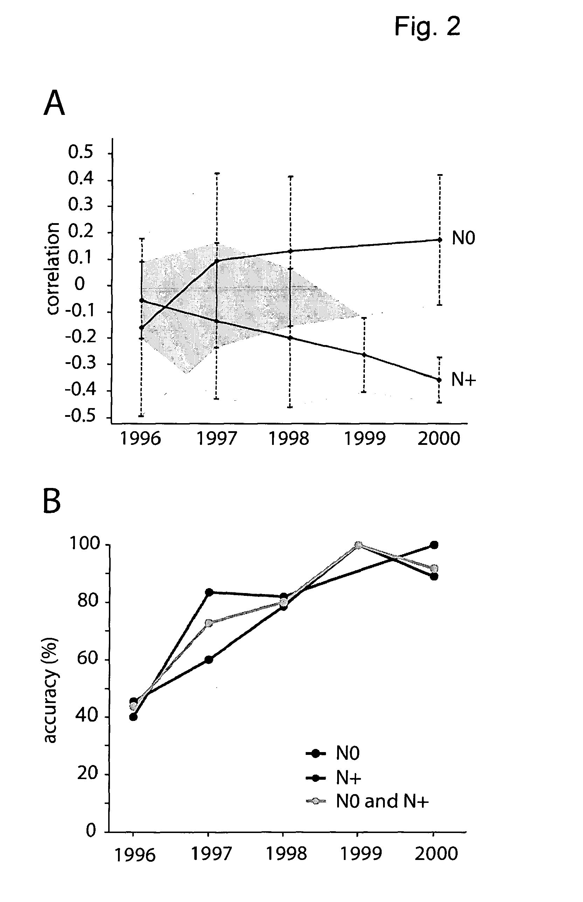

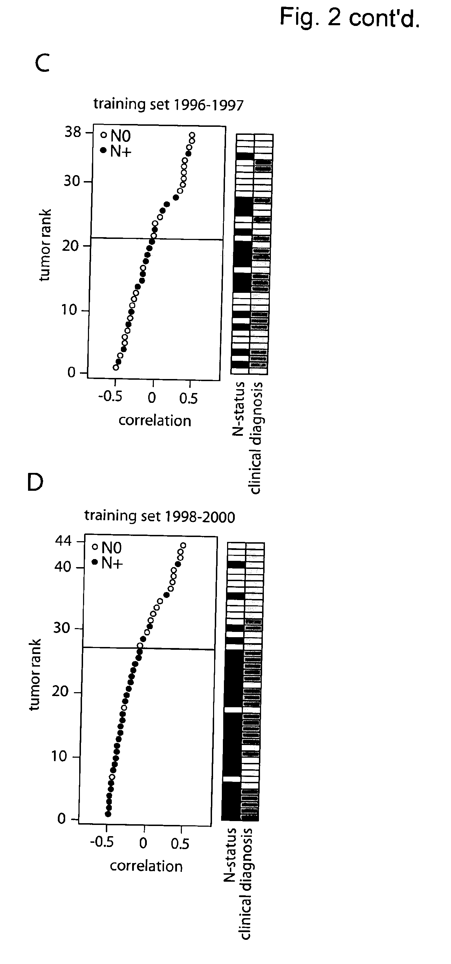

**Figure 1**. A predictor for HNSCC lymph node metastasis. **(a)** Expression profiles of the 102 predictor genes on the 82 primary tumor training set (middle). The predictor genes are clustered based on their similarities across the 82 tumors (Pearson around zero correlation, centroid clustering). Tumors are rank-ordered according to their correlation with the average N0 expression profile (left). The solid line represents the threshold for optimal overall accuracy. Tumors above the threshold show an expression profile that indicates that the patient is free of lymph node metastasis. In the right panel the patient's histological N-status, including the 3 year follow-up period, and the clinical diagnosis are shown (black indicates post-operative histological N+, white indicates post-operative histological N0, dark grey indicates clinical N+ and light grey indicates clinical N0 assessment). The asterix indicates a patient that developed lymph node metastasis post-treatment. **(b)** Expression profiles and tumor correlations from 6 training tumors samples (circles) and their technical replicates (squares). **(c)** as **(a)** only for a independent validation set of 22 primary HNSCC tumors. The threshold is set according to the optimal threshold established with the latter half of the training set **(Fig. 2d).**
**Figure 2.** Long-term tissue storage results in loss of predictive accuracy. (a) The mean correlation with the average no-metastasis profile and the standard deviation range for N0 patients (blue) and N+ patients (red) in the training set are shown for each year of surgery. **(b)** The N0 (blue), N+ (red) and overall (green) predictive accuracies increase from 40-45% for samples from 1996, to 89-100% for samples from 2000. **(c,d)** Correlation data from tumors with longer **(c)** or shorter **(d)** storage time. The predictor correctly predicts 22 of the 38 and 38 of the 44 samples, respectively.

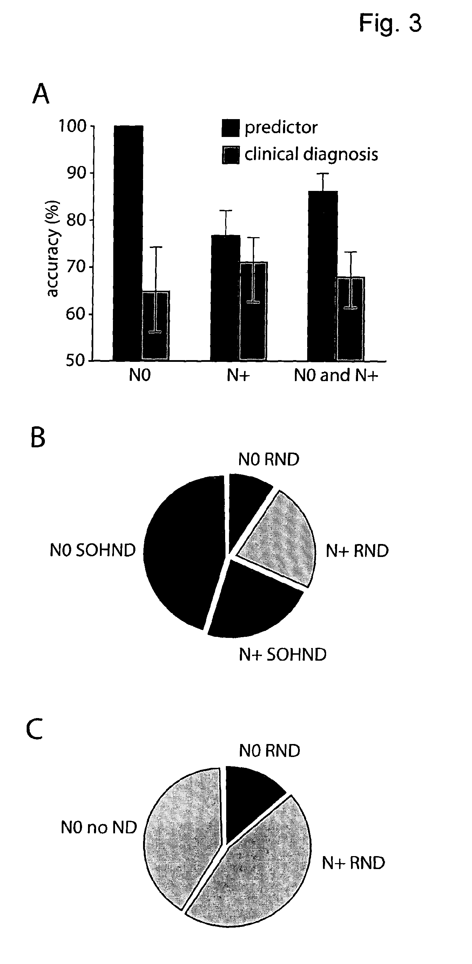

**Figure 3.** The predictor outperforms current clinical diagnosis on the validation set. (a) Predictive accuracies (PA) of current clinical diagnosis (blue) and the predictor (red) on the validation set. Error bars are based on the standard error for predictive accuracy. The predictor has a N0 PA of 100%, N+ PA of 77%, and overall PA of 86%. Clinical diagnosis has a N0 PA of 67%, N+ PA of 71%, and overall PA of 68%. **(b,c)** Treatment accuracy for the validation set, based on current clinical diagnosis **(b)** or if based on predictor outcome (c). Completely appropriate treatment is shown in green and under- or overtreatment in red. Current diagnosis resulted in 23% of patients receiving appropriate treatment (50% of N+ receiving an RND). Predictor based treatment would result in 86% of patients receiving appropriate treatment (75% of N0 that no longer receive any neck dissection and 100% of N+ receiving a RND).

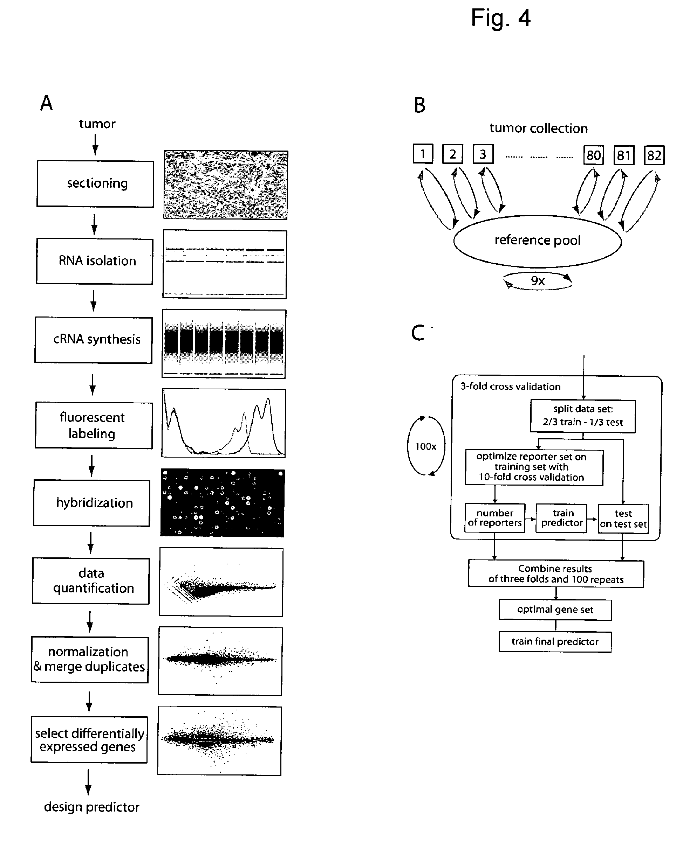

**Figure 4**. Study design and procedures overview. a, RNA was isolated from 2-3 tumor sections, followed by mRNA amplification and fluorescent labeling. After hybridization, scanned images were quantified and the data was normalized. Duplicates of each tumor were averaged and a predictor was designed using the differentially expressed genes. Quality control monitoring occurred after total RNA isolation, cRNA synthesis, labeling, scanning and normalization. b, The training experiment design involved 82 primary HNSCC tumors, compared in duplicate dye-swap against a common reference pool containing equal amounts of cRNA from each tumor. Nine reference pool self-self comparisons were generated in parallel, to establish an error-model for technical variation. c, The predictor was designed using a double loop training-validation protocol.

### DETAILED DESCRIPTION OF THE INVENTION

The inventors herein show that it is possible to give a more accurate prediction of the presence of lymph node metastasis of HNSCC than currently possible, by measuring mRNA expression of a concise set of genes (the predictor signature). It appeared possible to give an accurate prediction on basis of a set of 102 genes listed in Table I. It appeared that half of these genes have not been directly associated with tumorigenesis or metastasis before. Besides expected epithelial marker genes, interesting categories include genes (putatively) coding for extracellular matrix components, genes involved in cell adhesion including three members of the plakin family of cytolinkers and the enzyme transglutaminase 3, which play a role in maintaining tissue integrity; cell death genes: cell growth and maintenance genes and genes encoding hydrolyzing activities including proteins involved in degradation of the extracellular matrix (uPA and PAI-1) and a metalloproteinase. Another feature of the metastasis signature is that there is more down-regulation associated with metastasis (two thirds) than up-regulation. It is likely that this involves stromal and immune-regulatory components (Pollard, J.W. (2004) Nat. Rev. Cancer 4, 71-78; Chambers, A.F. *et al.* (2002) Nat. Rev. Cancer 2, 563-572). Many of the predictor genes belong to this categories, strengthening the argument for profiling bulk tumour tissue rather than laser-dissected regions densely populated with tumour cells.

It is shown herein that a diagnosis/prediction of the presence of metastases can be given using expression data of a set of only five genes from this large set of 102 genes. Table 2 indicates 15 of the genes which rank high in predictive value and which can especially be used to give a diagnosis or prediction of metastasis in HNSCC. Of course, accuracy of prediction will increase when more then five, preferably all 15 and even more preferably all 102 genes will be used on an array for gene expression analysis for the diagnostic/predictive signature.

Gene expression analysis is preferably done using a micro-array. The techniques for measuring and comparing gene expression on micro-arrays is well established within the art. It should be understood that it is not necessary to have the full length nucleotides encoding the above mentioned genes on said array: a stretch of nucleotides which is sufficient to establish unique hybridisation with the RNA expressed from said genes in the tumour cells can be used. Such a stretch of nucleotides is hereinafter referred to as 'element'. Preferably for the specific use of gene expression analysis for the current invention (i.e. with relation to detection of the presence of or the risk for metastases of HNSCC) such an array need not contain a large number of (different) genes or elements. It would be sufficient for the array to contain the necessary genes, as discussed above, and, preferably, some control genes, as will be discussed below. The array, which can be used for the analysis of the invention thus does not need to contain more than 1000 genes or elements, preferably not more than 500 genes or elements, more preferably not more than 200 genes or elements and most preferably from about 50 to about 150 genes or elements.

To investigate a gene expression profile the array should be subjected to hybridisation with target polynucleotide molecules from a clinically relevant source, in this case e.g. a person with HNSCC. Therefore, preferably a fresh frozen (within 1 hour from surgical removal), liquid nitrogen (at least -80 °C) stored tumour sample needs to be available. Said target polynucleotide molecules should be expressed RNA or a nucleic acid derived therefrom (e.g., cDNA or amplified RNA derived from cDNA that incorporates an RNA polymerase promoter). If the target molecules consist of RNA, it may be total cellular RNA, poly(A)⁺ messenger RNA (mRNA) or fraction thereof, cytoplasmic mRNA, or RNA transcribed from cDNA (cRNA). Methods for preparing total and poly(A)⁺ messenger RNA are well known in the art, and are described e.g. in Sambrook *et al.*, (1989) Molecular Cloning- A Laboratory Manual (2^{nd} Ed.) Vols. 1-3, Cold Spring Harbor, New York. In one embodiment, RNA is extracted from cells using guanidinium thiocyanate lysis followed by CsCl centrifugation (Chrigwin et *al.,* (1979) Biochem. 18:5294-5299). In another embodiment, total RNA is extracted using a silica-gel based column, commercially available examples of which include RNeasy (Qiagen, Valencia, CA, USA) and StrataPrep (Stratagene, La Jolla, CA, USA). Poly(A)⁺ messenger RNA can be selected, e.g. by selection with oligo-dT cellulose or, alternatively, by oligo-dT primed reverse transcription of total cellular RNA. In another embodiment, the polynucleotide molecules analyzed by the invention comprise cDNA, or PCR products of amplified RNA or cDNA.

Preferably, the target polynucleotides are detectably labelled at one or more nucleotides. Any method known in the art may be used to detectably label the nucleotides. Preferably, this labelling incorporates the label uniformly along the length of the polynucleotide and is carried out at a high degree of efficiency. One embodiment for this labelling uses oligo-dT primed reverse transcription to incorporate the label; however, conventional methods hereof are biased toward generating 3' end fragments. Thus, in this embodiment, random primers (e.g. 9-mers) are used in reverse transcription to uniformly incorporate labelled nucleotides over the full length of the target polynucleotides. Alternatively, random primers may be used in conjunction with PCR methods or T7 promoter-based *in*, *vitro* transcription methods in order to amplify the target polynucleotides.

In a preferred embodiment, the detectable label is a luminescent label. For example, fluorescent labels, bioluminescent labels, chemiluminescent labels and colorimetric labels may be used. In a highly preferred embodiment, the label is a fluorescent label, such as a Cy5 or Cy3 , fluorescein, a phosphor, a rhodamine, or a polymethine dye or derivative. In another embodiment, the detectable label is a radiolabeled nucleotide.

The array may be any nucleotide array which represents five or more of the genes of Table 2 or Table 1. Presence of other genes on the array is allowable and the expression data from such other genes need not neccessarily be considered for the present application.. These arrays can be commercially available (e.g. from Agilent US; Affymetrix Inc, CA, USA; and others) or can be self-made by spotting or synthesizing nucleotides which are known to selectively hybridise to the target genes on a surface. Methods to prepare such arrays are well within the skill of the artisan. The microarrays can comprise cDNA, but can also comprise short oligonucleotides (Affimatrix and Nimblegen) or long oligonucleotides which are synthesized *in situ*_ (Agilent); in another embodiment the arrays comprise long oligonucleotides and are self-made by spotting.

Nucleic acid hybridisation and wash conditions are chosen so that the target polynucleotide molecules specifically hybridize to the complementary polynucleotide sequences of the array, preferably to a specific array site, wherein its complementary DNA is located. Optimal hybridisation conditions will depend on the type (e.g., RNA or DNA) of the target nucleotides and array. General parameters for specific (i.e., stringent) conditions of hybridisation are described in Sambrook *et al. (supra).* Typical hybridisation conditions for cDNA microarrays are hybridisation in 5 X SSC plus 0.2% SDS at 65 °C four hours, followed by washes at 25 °C in low stringency wash buffer (1 X SSC plus 0.2% SDS), followed by 10 minutes at 25 °C in higher stringency wash buffer (0.1 X SSC plus 0,2% SDS).

When fluorescently labelled probes are used, the fluorescence emissions at each site of the microarray may be detected by scanning confocal laser microscopy. In one embodiment, the arrays is scanned with a laser fluorescent scanner with a computer controlled X-Y stage and a microscope objective. Fluorescent laser scanning devices are described in e.g. Schena *et al*. (1996) Genome Res. 6:639-645. Signals are recorded and, in a preferred embodiment, analysed by computer using a 12 or 16 bit analog to digital board. In one embodiment the scanned image is despeckled using a graphics program (e.g., Hijaak Graphics Suite) and then analysed using an image gridding program that creates a spreadsheet of the average hybridisation at each wavelength at each site.

Not all of the genes are evenly contributing to the discriminating effect. As is shown in Table 1. the genes differ in significant expression. Although the statistical data presented in the Examples are calculated with all of the 102 genetic elements of Table 1, it is submitted that a good distinction between the two groups of patients and therewith a good diagnosing/predicting ability of the signature gene set can also be achieved with only a part of the elements of Table 1. At least 5 (5%) of the elements of Table 1 are included in the analysis, more preferably 20%, more preferably 40%, more preferably 60%, more preferably 80%, more preferably 90% and most preferably all of the elements. It would be advisable not to randomly choose the elements, but to pick the most discriminating genes in this list. Table 2 gives an overview of the top 15 genes out of the 102 genes of table 1, of which at least 5, more preferably at least 6, more preferably at least 7, more preferably at least 8, more preferably at least 9, more preferably at least 10, more preferably at least 11, more preferably at least 12, more preferably at least 13, more preferably at least 14, and most preferably all 15 can be used for making up the signature with which the microarray analysis is performed.

As indicated above, various combinations of these genes can be used for determining the presence of lymph node metastases in several ways.

On dual channel DNA microarrays this is performed by determining the expression level ratios of the genes in the primary tumour sample versus expression of the same genes in reference material. The reference material can be derived from a pool of total RNA or amplified mRNA from a set of HNSCC primary tumours with established lymph node metastasis characteristics. The individual gene expression ratios contribute towards the expression ratio signature of a sample. The degree of correlation of a sample's signature with the signatures of samples with known metastatic status (preferably calculated by the cosine correlation (Jones, W. P., & Furnas, G. W. (1987). Pictures of Relevance: A Geometric Analysis of Similarity Measures. Journal of the American Society for Information Science, 36 (6), 420-442) as, e.g, provided by the Genesis software; http://genome.tugraz.at/Software/Genesis/Description.html) is used to predict the metastatic state of the unknown sample. The correlation threshold for predicting the metastatic state is based on the optimal threshold for discriminating between the metastatic states of the samples with known metastatic states, which can easily be determined by a person skilled in the art

Other measurements of absolute expression and expression ratios of these genes can also be used. Reference material can be derived from other sources than a pool of samples with known metastatic states. Preferably, however, samples with known metastatic states are still required to determine the correlation threshold for determining the metastatic status.

Expression ratios can also be derived from single channel microarray experiments, using as a reference so-called housekeeping genes (i.e. with stable expression across many different samples) or a collection of housekeeping genes or any collection of genes or features with stable expression. Again here it is preferred to use samples with known metastatic states to determine the correlation threshold for determining the metastatic status.

Gene expression measurements and the derived ratios can also be obtained by (quantitative) reverse transcription PCR or any other assay for gene expression, using as a reference any gene or collection of genes that have stable expression across many samples. In a specific embodiment of this application of the invention, samples with known metastatic states are still required to determine the correlation threshold for determining the metastatic status.

In the absence of tumour samples with known metastatic states for calibration of the prediction, the genes or various combinations of the (expression analysis of the) genes can still be used to predict the metastatic state. In these embodiments of the invention an absolute or relative measurement of gene expression is determined for example using single or dual channel DNA microarrays, or by other methods such as (quantitative) reverse transcription PCR. Increased expression of the genes in table 1 or 2 with a positive N+ correlation will hereby contribute positively towards prediction of the N+ status and negatively towards prediction of the N0 status. Conversely, increased expression of the genes in table 1 or 2 with a negative N+ correlation will contribute positively towards N0 prediction and negatively towards N+ prediction. Increased expression in both cases indicates an increase relative to a suitable marker gene or feature, set of genes or features or collectively in relation to each other.

However, a person skilled in the art is able to obtain the reference data that have been produced in the below example, since this data is available as dataset E-UMCU-11 from the public micro-array database ArrayEcpress (http://www.ebi.ac.uk/arrayexpress/). When desiring to predict or determine the presence of metastases for a certain patient, the practitioner should take a biopsy from that patient, isolate the RNA and determine the expression of at least 5 of the elements of Table 1. To normalize these expression data with respect to the data of the reference set E-UMCU-11, it is possible to correct the data for variations with the help of expression data of a control gene or element which is not affected by the tumour state (such as a housekeeping gene), which is present in the reference set E-UMCU-11 and should also be available on the array that has been used to determine the expression profile of the patient to be assessed. In stead of one control gene or element, also the mean value of a poll of control genes or elements can be taken. This correction can, for instance, be done by subtracting the expression level of the control gene(s)/element(s) from the expression levels of each of the tested genes/elements. Preferably, the ratio for every tested gened with respect to the control gene(s) is calculated for both the patient's expression profile as well as for the expression data of the reference set.

With these figures, the correlation with the mean value of the N0 values of the reference set should be calculated. If this correlation is negative (i.e. a value below zero) it can be concluded that the patient is N+ (i.e. having or prone to develop metastases). Conversely, the correlation can be calculated with respect to the mean value of the N+ values of the reference set. Then a negative correlation indicates a match with the N0 group.

Further enablement for a diagnosis/prediction of cancer metastasis on basis of gene expression analyses can be found in WO 03/010337, indicating that methods as have been generally described above are well within the skills of the practitioners in the art.

### EXAMPLE

### Data accessibility

MIAME¹ compliant data in MAGE-ML² format as well as complete descriptions of protocols, microarrays and clinical parameters have been submitted to the public microarray database ArrayExpress (http://www.ebi.ac.uk/arrayexpress/) with the following accession numbers: Microarray layout, A-UMCU-3; HNSCC tumour data, E-UMCU-11; Protocols for sectioning of tumour material, P-UMCU-18; RNA isolation, P-UMCU-19; DNase treatment, P-UMCU-20; mRNA amplification, P-UMCU-21; generating reference pool, P-UMCU-26; cRNA labeling, P-UMCU-22; hybridization and washing of slides, P-UMCU-23 and P-UMCU-24; scanning of slides, P-UMCU-25; Image analysis, P-UMCU-11

### Tumor samples

For the training set, 92 samples were randomly taken from a collection of primary tumours surgically removed between 1996 and 2000 and that fulfilled the following criteria: biopsy-proven HNSCC in the oropharynx and oral cavity; no previous malignancies in the head and neck region; tumour sections contained more than 50% tumour cells. Of these 92 tumours, 82 passed total RNA and cRNA quality control (QC) and were included in this study. For the validation set, 27 tumours were randomly taken from the same collection of tumours, surgically removed between 2000 and March 2001, and that fulfilled the same selection criteria. Of these, 22 passed total RNA and cRNA QC and were included in this study. The diagnostic procedures for clinical staging of cervical lymph nodes was performed according to the Netherlands national guidelines for oral cavity and oropharynx carcinomas, by clinical examination (palpation) of the neck region, followed by bilateral ultrasound examination, computed tomography (CT) and/or magnetic resonance imaging (MRI). Suspected nodes were subjected to aspiration cytology. In this way, patients were preoperatively classified as either N0 or N+, the latter in the case of aspirates yielding metastatic tumour cells. Only in the case of obvious neck involvement, as shown by huge swelling, were the patients classified as N+ without additional efforts to prove the presence of metastasis.
Surgery was aimed at complete tumour removal. With regard to the neck, in the case of clinical N0 only a SOHND was performed³. In cases clinically classified as N+ a RND was performed including all five lymph node levels³. Postoperative irradiation was administered in accordance with current practice and depending on margin status, tumour growth features, number of positive nodes and extracapsular growth. In practice, 36 out of 60 clinically assessed N0 patients and 38 out of 43 clinically assessed N+ patients received radiation therapy. This treatment as well as additional clinical information is presented in Supplemental data 2 (for accessibility, see above). After surgery, patients were periodically checked for development of neck metastasis, and patients initially classified as N0 but showing positive nodes in their surgical specimen or developing neck nodes within a time span of 3 years after surgery without having another head and neck cancer that could be responsible for this metastasis, were retrospectively added to the N+ patient group. Less than 5% of patients with HNSCC in the oral cavity or oropharynx subsequently develop metastasis after treatment^{4,5}. Here, for the training and validation cohorts, one patient subsequently developed positive neck nodes after surgery. Three years is to be considered as a reliable time period, since at least 80% of the recurrences are known to take place in the first two years after surgery (Takes, R.P. *et al.* (2001) J Pathol 194,298-302 ; Jones, K.R., *et al.,* (1992) Arch. Otolaryngol. Head Neck Surg. 118, 483-485)
Fresh tumour tissue was taken from the surgical specimen, snap-frozen in liquid nitrogen immediately after surgical removal and stored at -80°C. Frozen sections were cut for RNA isolation and immediately transferred to a RNAlater solution (Ambion). A haematoxylin and eosin stained section was prepared for tumour percentage assessment. Only samples with at least 50 percent tumour cells were used. For a small number of samples the tumour percentage was increased by removing areas with no tumour cells.

### RNA isolation

Total RNA was isolated from 2-3 sections (20 µm) with TRIzol reagent (Invitrogen), followed by a purification using the RNeasy mini-kit (Qiagen) and a DNase treatment using the Qiagen DNA-free kit. The yield and quality of total RNA was checked by spectrophotometry and by the Agilent 2100 Bioanalyser (Agilent). Total RNA quality control criteria were in accordance with the Tumour Analysis Best Practices Working Group⁶, discarding samples with no clear 18S and 28S ribosomal bands. We also removed samples that had a yield lower than 500 ng total RNA or showed mycoplasma contamination.

### cRNA synthesis and labeling

mRNA was amplified by in *vitro* transcription using T7 RNA polymerase on 1 µg of total RNA. First a double stranded cDNA template was generated including the T7 promoter. Next, this template was used for *in vitro* transcription with the T7 megascript kit (Ambion) to generate cRNA. During the *in vitro* transcription, 5-(3-aminoallyl)-UTP (Ambion) was incorporated into the single-stranded cRNA. The yield and quality of the cRNA was analyzed by spectrophotometry and by the Agilent 2100 Bioanalyser. Samples with a yield less than 5000 ng or with small cRNA fragments (median less than 500 bp) were not used.
Cy3 or cy5 fluorophores (Amersham) were coupled to 500 ng of cRNA. After coupling, free dye molecules were removed using Clontech ChromoSpin-30 columns (Clontech). The yield and label incorporation (5-7%) of the cy-labeled cRNA was checked using spectrophotometry. Before hybridization, 300 ng of cy-labeled cRNA from one tumor was mixed with an equal amount of reverse color cy-labeled material from the reference sample.

### Microarray production

The Human Array-Ready Oligo set (version 2.0) was purchased from Qiagen and printed on Corning UltraGAPS slides as described elsewhere⁷. The microarrays contained 70-mer oligonucleotides representing 21,329 genes as well as 3871 additional features for control purposes.

### Microarray hybridization

Before use, the microarray slides were treated with sodium-borohydrate solution to reduce auto-fluorescence in the cy3-channel⁸. The labelled cRNA targets were hybridized on the microarray for 10 hours at 42 °C using the Ventana Discovery Hybridization Station in combination with the ChipMap-80 Kit (Ventana Europe). After hybridization the slides were manually washed and scanned in the Agilent G2565AA DNA Microarray Scanner (100% laser power, 30% PMT).

### Pre-processing of expression data

The scanned images were quantified and background corrected using Imagene 4.0 software (Biodiscovery). The expression data was normalized for dye and print-tip biases using a Lowess per print-tip normalization algorithm⁹ applied in the statistical package R¹⁰. Following normalization, variance stabilization (VSN)¹¹ was applied to stabilize variance in the intensity data. Both duplicate dye-swap hybridizations of each tumour were averaged and for each gene a tumour-reference ratio was calculated. Reference versus reference hybridizations were used to build a gene error model for technical variation. Nine reference self-self comparisons were performed in dye-swap (18 hybridizations), resulting in nine reference ratios for each gene on the microarray. These nine reference ratios yield an estimate of the technical variation for each gene. To test whether a gene in a tumour samples shows differential expression, a Student's t-test was applied on the tumour ratio and the corresponding nine reference ratios (technical variation). The calculated p-values for differential expression were used to select those genes that show differential expression in the tumour samples.

### Supervised classification

A classifier was constructed to distinguish between N0 and N+ patients. Of the 21,329 genes on the microarray, 6221 were excluded based on aberrant signal and spot morphology in one of the 164 hybridizations. From these remaining 15,108 genes, only genes that were significantly different from the reference in at least 31 tumours were selected based on the error model for technical variation (p<0.01). This resulted in a set of 1,986 genes. For these genes the signal-to-noise-ratio (SNR)¹² was computed and employed to rank the genes (top ranked genes being genes that are best suited to distinguish the outcome classes). The optimal gene set to employ in the classifier (a nearest mean classifier similar to the classifier employed in¹³), was determined by gradually expanding the gene set starting from the highest ranked gene. At each expansion round the nearest mean classifier¹³ was trained on a training set and tested on a test set. The performance on the test set served as a quality measure of the gene set. The performance was measured as the average of the false positive (N0 classified as N+) and false negative (N+ classified as N0) rates of the test samples. Initially the performance increases as the set is expanded. The expansion of the gene set is terminated when the performance deteriorates, i.e. when the optimal performance is reached. The steps of ranking the genes and training and testing the classifier are performed in a 10-fold cross-validation procedure. The output of this procedure is an optimal number of top-ranked genes and a trained classifier. To ensure independent validation, this process of optimizing the set of genes and training the classifier is wrapped in a second 3-fold cross-validation loop. This entails that the optimization of the gene set and the training of the classifier is performed on 2/3 of the data, while the classifier is validated on 1/3 of the data. Since this 1/3 of the data is never involved in any of the gene selection and training steps, this ensures completely independent validation of the classifier, which avoids selection bias^{14,15} and therefore results in a reliable performance estimate. This double-loop procedure determined 102 genes to form the final diagnostic classifier. This classifier was trained on the complete set of 82 samples by recalculating the signal-to-noise ratio for all genes and subsequently selecting the top 102 genes. The predictor was trained using the 102 selected genes and the 82 training samples. A decision threshold for this classifier was fixed such that the highest overall predictive accuracy for both N0 and N+ tumours was reached.

### Statistics

Odds ratios (OR) were calculated by fitting a logistic regression model on the prediction outcome of the validation set. The predictor had an infinitive OR since no false negative prediction was made. To get an estimate of the OR for the predictor, one false negative was artificially introduced resulting in a predictor OR of 30 (p=0.006) and a clinical OR of 4.2 (p=0.15). The standard error for predictive accuracy (Fig. 3a) includes the predictions made on the latter half of the training set.

### References

1. Brazma, A. *et al.* Minimum information about a microarray experiment (MIAME)-toward standards for microarray data. *Nat. Genet.* 29, 365-371 (2001).
2. Spellman. P. T. *et al.* Design and implementation of microarray gene expression markup language (MAGE-ML). *Genome Biol.* 3, RESEARCH0046 (2002).
3. Robbins, K. T. *et al.* Neck dissection classification update: revisions proposed by the American Head and Neck Society and the American Academy of Otolaryngology-Head and Neck Surgery. *Arch. Otolaryngol. Head Neck Surg.* **128**, 751-758 (2002).
4. Carvalho, A. L., Kowalski, L. P., Borges, J. A., Aguiar, S., Jr. & Magrin, J. Ipsilateral neck cancer recurrences after elective supraomohyoid neck dissection. *Arch. Otolaryngol. Head Neck Surg.* **126,** 410-412 (2000).
5. Ridge, J. A. Squamous cancer of the head and neck: surgical treatment of local and regional recurrence. *Semin. Oncol.* 20, 419-429 (1993).
6. Expression profiling-best practices for data generation and interpretation in clinical trials. *Nat. Rev. Genet.* 5, 229-237 (2004).
7. van de Peppel, J. *et al.* Monitoring global messenger RNA changes in externally controlled microarray experiments. *EMBO Rep.* 4, 387-393 (2003).
8. Raghavachari, N., Bao, Y. P., Li, G., Xie, X. & Muller, U. R. Reduction of autofluorescence on DNA microarrays and slide surfaces by treatment with sodium borohydride. *Anal. Biochem.* **312**, 101-105 (2003).
9. Yang, Y. H. *et al.* Normalization for cDNA microarray data: a robust composite method addressing single and multiple slide systematic variation. *Nucleic Acids Res.* **30**, e15 (2002).
10. Ihaka, R. & Gentleman, R. R: a language for data analysis and graphics. J. *Comp. Graph. Statist.* 5, 299-314 (1996).
11. Huber, W.. von Heydebreck, A., Sultmann, H., Poustka, A. & Vingron, M. Variance stabilization applied to microarray data calibration and to the quantification of differential expression. *Bioinformatics* 18 Suppl 1, S96-S104 (2002).
12. Golub, T. R. *et al.* Molecular classification of cancer: class discovery and class prediction by gene expression monitoring. *Science* **286**, 531-537 (1999).
13. van 't Veer, L. J. *et al.* Gene expression profiling predicts clinical outcome of breast cancer. *Nature* **415**, 530-536 (2002).
14. Simon, R., Radmacher, M. D., Dobbin, K. & McShane, L. M. Pitfalls in the use of DNA microarray data for diagnostic and prognostic classification. *J. Natl. Cancer Inst.* **95,** 14-18 (2003).
15. Ambroise, C. & McLachlan, G. J. Selection bias in gene extraction on the basis of microarray gene-expression data. *Proc Natl Acad Sci U S A* **99**, 6562-6566 (2002).

**Table 1. Complete list of the 102 HNSCC predictor genes**

| **Gene** | **GenBank ID** | **Gene name** | **N+ corr** |
|---|---|---|---|
| FTH1 | NM_002032 | ferritin, heavy polypeptide 1 | 0.726 |
| COL5A1 | NM_000093 | COL5A1 protein (collagen 5 type A1 like) | 0.627 |
| NCOR2 | NM_006312 | Nuclear receptor co-repressor 2 | 0.617 |
| P4HA1 | NM_000917 | proline 4-hydroxylase alpha polypeptide I | 0.572 |
| TNFAIP3 | NM_006290 | tumour necrosis factor, alpha-induced protein 3 | 0.536 |
| PLAU | NM_002658 | urokinase plasminogen activator (uPA) | 0.535 |
| COL5A3 | NM_015719 | Collagen, type V, alpha 3 | 0.521 |
| SPOCK | NM_004598 | Sparc/osteonectin, cwcv and kazal-like domains proteoglycan (testican) | 0.500 |
| FAP | NM_004460 | fibroblast activation protein, alpha | 0.498 |
| ADAM12 | NM_003474 | a disintegrin and metalloproteinase domain 12 (meltrin alpha) | 0.482 |
| TPM2 | NM_003289 | tropomyosin 2 (beta) | 0.470 |
| MICAL2 | NM_014632 | flavoprotein oxidoreductase MICAL2 | 0.459 |
| D2S448 | XM_056455 | D2S448 (Melanoma associated gene) | 0.446 |
| PAI-1 | NM_000602 | plasminogen activator inhibitor type 1 | 0.440 |
| REN | NM_000537 | renin | 0.383 |
| POSTN | NM_006475 | periostin, osteoblast specific factor | 0.367 |
| CKTSF1B1 | NM_013372 | cysteine knot superfamily 1, BMP antagonist 1 (DRM/GREMLIN) | 0.330 |
| IER3 | NM_003897 | immediate early response 3 | 0.303 |
| MMD | NM_012329 | monocyte to macrophage differentiation-associated | 0.300 |
| CTDSP1 | NM_021198 | CTD (carboxy-terminal domain, RNA polymerase II, polypeptide A) small phosphatase 1 | 0.260 |
| PSMD2 | NM_002808 | proteasome (prosome, macropain) 26S subunit, non-ATPase, 2 | 0.256 |
| MAN1B1 | NM_016219 | mannosidase, alpha, class 1B, member 1 | 0.254 |
| DKK3 | NM_013253 | dickkopf homolog 3 (Xenopus laevis) | 0.140 |
| NT5C3 | NM_016489 | 5'-nucleotidase, cytosolic III | 0.138 |
| DAPK3 | NM_001348 | death-associated protein kinase 3 | 0.022 |
| NDUFB4 | NM_004547 | NADH dehydrogenase (ubiquinone) 1 beta subcomplex, 4, 15kDa | 0.012 |
| UBA52 | NM_003333 | ubiquitin A-52 residue ribosomal protein fusion product 1 | -0.002 |
| C9orf5 | NM_032012 | chromosome 9 open reading frame 5 | -0.075 |
| COPG | NM_016128 | Coat protein gamma-cop | -0.081 |
| RGS5 | NM_003617 | regulator of G-protein signalling 5 | -0.097 |
| FLJ12236 | AK022298 | Homo sapiens cDNA FLJ12236 fis, clone MAMMA1001244 | -0.104 |
| IDI1 | NM_004508 | Isopentenyl-diphosphate delta isomerase | -0.126 |
| RPL37A | NM_000998 | ribosomal protein L37a | -0.162 |
| ZDHHC18 | NM_032283 | Zinc finger DHHC domain containing protein 18 | -0.175 |
| MO30 | M26463 | Homo sapiens immunoglobulin mu chain antibody MO30 (IgM) mRNA, complete cds | -0.194 |
| FLJ20073 | NM_017654 | Hypothetical protein FLJ20073 | -0.215 |
| FLJ30814 | AK055376 | Homo sapiens cDNA FLJ30814 fis, clone FEBRA2001529 | -0.222 |
| PLK2 | NM_006622 | polo-like kinase 2 | -0.230 |
| MMPL1 | NM_004142 | Matrix metalloproteinase-like 1 | -0.230 |
| | Z95126 | Human DNA sequence from clone RP1-30P20 on chromosome Xq21.1-21.3 | -0.232 |
| BAL | NM_031458 | B aggressive lymphoma protein (BAL) | -0.234 |
| OSBP2 | NM_030758 | Oxysterol binding protein 2 | -0.244 |
| PARVB | NM_013327 | Parvin, beta | -0.286 |
| CEBPA | NM_004364 | CCAAT/enhancer binding protein (C/EBP), alpha | -0.290 |
| ZNF533 | NM_152520 | zinc finger protein 533 | -0.291 |
| ABCA12 | NM_015657 | ATP-binding cassette, sub-family A (ABC1), | -0.293 |
| | | member 12 | |
| LOR | NM_000427 | loricrin | -0.322 |
| E2F5 | NM_001951 | E2F transcription factor 5, p130-binding | -0.356 |
| APM2 | NM_006829 | Adipose specific 2 | -0.360 |
| CAPNS2 | NM_032330 | CAPNS2 (calpain small subunit 2) | -0.360 |
| MGC13219 | NM_032931 | Hypothetical protein MGC13219 | -0.367 |
| FLJ22202 | NM_024883 | Hypothetical protein FLJ22202 | -0.383 |
| KRT23 | NM_173213 | keratin 23 (histone deacetylase inducible) | -0.409 |
| PPT2 | NM_005155 | palmitoyl-protein thioesterase 2 | -0.417 |
| PGBD5 | NM_024554 | piggyBac transposable element derived 5 | -0.469 |
| SSH2 | NM_033389 | SSH2 (slingshot 2) | -0.475 |
| ALOX12B | NM_001139 | arachidonate 12-lipoxygenase, 12R type | -0.480 |
| MAL2 | NM_052886 | mal, T-cell differentiation protein 2 | -0.544 |
| ZD52F1 | NM_033317 | Hypothetical gene ZD52F1 | -0.562 |
| EPPK1 | AB107036 | Epiplakin 1 | -0.566 |
| S100A7 | NM_002963 | S100 calcium binding protein A7 (psoriasin 1) | -0.580 |
| FLJ22184 | NM_025094 | Hypothetical protein FLJ22184 | -0.586 |
| MAP17 | NM_005764 | membrane-associated protein 17 (MAP17) | -0.591 |
| FLJ13497 | AK023559 | Homo sapiens cDNA FLJ13497 fis, clone PLACE14518 | -0.603 |
| ECM1 | NM_022664 | extracellular matrix protein 1 | -0.610 |
| TGM3 | NM_003245 | transglutaminase 3 (E polypeptide, protein-glutamine-gamma-glutamyltransferase) | -0.629 |
| RAD17 | NM_133344 | RAD17 homolog (S. pombe) | -0.631 |
| FLJ30988 | AK055550 | Homo sapiens cDNA FLJ30988 fis, clone HLUNG 1000030 | -0.650 |
| C10orf26 | NM_017787 | chromosome 10 open reading frame 26 | -0.653 |
| PALM2 | NM_053016 | paralemmin 2 | -0.655 |
| C4.4A | NM_014400 | GPI-anchored metastasis-associated protein homolog (C4.4A) | -0.665 |
| ECG2 | NM_032566 | Esophagus cancer-related gene-2 protein precursor (ECRG-2) | -0.670 |
| PPL | NM_002705 | periplakin | -0.672 |
| HPCAL1 | NM_002149 | hippocalcin-like 1 | -0.674 |
| SLPI | NM_003064 | secretory leukocyte protease inhibitor (antileukoproteinase) | -0.677 |
| PI3 | NM_002638 | protease inhibitor 3, skin-derived (SKALP) | -0.680 |
| FLJ25911 | AK098777 | Hypothetical protein FLJ25911 [Fragment] | -0.680 |
| CLIC3 | NM_004669 | chloride intracellular channel 3 | -0.691 |
| BENE | NM_005434 | BENE protein | -0.698 |
| FLJ00074 | AK024480 | FLJ00074 protein [Fragment] | -0.699 |
| DKFZp547F 134 | AL512697 | Homo sapiens mRNA; cDNA DKFZp547F134 (from clone DKFZp547F134) | -0.706 |
| PLA2G4B | NM_005090 | phospholipase A2, group IVB (cytosolic) | -0.707 |
| | AF339799 | Homo sapiens clone IMAGE:2363394, mRNA sequence | -0.708 |
| TRGV9 | BC062761 | T cell receptor gamma variable 9 | -0.710 |
| DSG3 | NM_001944 | desmoglein 3 (pemphigus vulgaris antigen) | -0.711 |
| FLJ12787 | NM_032175 | Hypothetical protein FLJ12787 | -0.734 |
| LLGL2 | NM_004524 | lethal giant larvae homolog 2 (Drosophila) | -0.738 |
| SMC5L1 | NM_015110 | SMC5 structural maintenance of chromosomes 5-like 1 | -0.741 |
| ODCP | NM_052998 | Ornithine decarboxylase-like protein (EC 4.1.1.17) (ODC-paralogue) (ODC-p) | -0.741 |
| FLJ31161 | AK055723 | Homo sapiens cDNA FLJ31161 fis, clone KIDNE 1000028 | -0.747 |
| FLJ21214 | AK024867 | Homo sapiens cDNA: FLJ21214 fis, clone COL00523 | -0.748 |
| SPINK5 | NM_006846 | serine protease inhibitor, Kazal type, 5 | -0.749 |
| KIAA0350 | XM_290667 | KIAA0350 protein | -0.760 |
| PGLYRPL | NM_052890 | peptidoglycan recognition protein L precursor | -0.764 |
| S100A9 | NM_002965 | S 100 calcium binding protein A9 (calgranulin B) | -0.773 |
| DNAH11 | NM_003777 | Dynein, axonemal, heavy chain-11 | -0.776 |
| LAGY | NM_032495 | lung cancer-associated Y protein; homeodomain-only protein | -0.793 |
| IVL | NM_005547 | involucrin | -0.801 |
| TNFRSF5 | NM_001250 | tumor necrosis factor receptor superfamily, member 5 (CD40) | -0.802 |
| SRP19 | NM_003135 | signal recognition particle 19kDa | -0.814 |
| KLK12 | NM_019598 | kallikrein 12 | -0.837 |
| IL22RA1 | NM_021258 | interleukin 22 receptor, alpha 1 | -0.885 |

**TABLE 2**

| **Gene** | **GenBank ID** | **Gene name** | **N+ corr.** |
|---|---|---|---|
| COL5A3 | NM_015719 | Collagen, type V, alpha 3 | 0.520719 |
| COL5A1 | NM_000093 | COL5A1 protein (collagen 5 type A1 like) | 0.626949 |
| ZD52F1 FLJ2591 | NM_033317 | Hypothetical gene ZD52F1 | -0.56185 |
| 1 | AK098777 | Hypothetical protein FLJ25911 [Fragment] | -0.6799 |
| EPPK1 KIAA03 | AB107036 | Epiplakin 1 | -0.56641 |
| 50 DNAH1 | XM_290667 | KIAA0350 protein | -0.76021 |
| 1 | NM_003777 | Dynein, axonemal, heavy chain-11 | -0.77599 |
| PI3 | NM_002638 | protease inhibitor 3, skin-derived (SKALP) procollagen-proline, 2-oxoglutarate 4-dioxygenase (proline 4-hydroxylase), alpha | -0.67983 |
| P4HA1 | NM_000917 | polypeptide I Ornithine decarboxylase-like protein (EC | 0.572479 |
| ODCP | NM_052998 | 4.1.1.17) (ODC-paralogue) (ODC-p) monocyte to macrophage differentiation- | -0.74146 |
| MMD | NM_012329 | associated | 0.299881 |
| TPM2 | NM_003289 | tropomyosin 2 (beta) | 0.470038 |
| SRP19 | NM_003135 | signal recognition particle 19kDa | -0.81361 |
| IL22RA1 FLJ3116 | NM_021258 | interleukin 22 receptor, alpha 1 Homo sapiens cDNA FLJ31161 fis, clone | -0.88482 |
| 1 | AK055723 | KIDNE 1000028 | -0.7475 |

## Claims

1. A nucleotide array for the detection of metastasis in head and neck squamous cell cancer (HNSCC) comprising at least 5 of the elements of Table 1, more preferably 70%, more preferably 80%, more preferably 90%, more preferably 95%, more preferably 99% and most preferably all.

2. A nucleotide array for the detection of metastasis in HNSCC having one or more of the elements of the genes listed in Table 2.

3. A method to establish reference and control gene expression profiles of patients having had metastasis after HNSCC (N+ group) or no metastasis after HNSCC (N0 group) by analysing the gene expression from a tumour biopsy sample of each patient, or from pooled samples of each group of patients, on an array according to claim 1 or 2.

4. A method to predict the presence or risk on occurrence of lymph node metastasis of a HNSCC patient. comprising:
a. taking a biopsy sample from the tumour of the patient;
b. isolating the nucleic acid from the biopsy sample;
c. analyse the gene expression profile of said nucleic acid by assaying it with a nucleotide array according to claim 1 or 2;
d. classifying the expression profile as N+ or N0 by determining whether the expression profile would match the expression profile of a group of HNSCC patients known to have developed metastasis.

5. A method according to claim 3 or 4, where the biopsy sample is a fresh biopsy sample.

6. A method according to claim 4, wherein the analysis of the gene expression profile comprises:
a. hybridising the nucleic acid form the biopsy sample with the nucleotide array according to claim 1 or 2;
b. determining the amount of hybridisation of each of the elements of the nucleotide array relative to the amount of hybridisation of each element with a reference sample, said step optionally involving a normalisation step;
c. determining for each element of the array whether the expression of the corresponding gene in the biopsy sample is more or less than the expression of the corresponding gene in the reference sample.

7. A method according to claim 4 or 6, wherein the expression profile is classified as N+ (high risk of metastasis) or N0 (low or no risk of metastasis) according to the steps of:
a. determining the collective correlation of the classifier/predictor genes or elements present in the expression profile with the average N+ or N0 profile from primary tumors with previously established N-status; and
b. determining the predictive threshold based on the correlation threshold from primary tumors with previously established N-status

8. A method according to any of claims 4, 6 or 7, wherein the gene expression profile of a group of HNSCC patients known to have developed metastasis is the expression profile contained in the dataset E-UMCU-11, available in the public microarray database ArrayExpress (http://www.ebi.ac.uk/arrayexpress/).

9. A method according to claims 7 or 8, wherein the correlation is determined using the cosine correlation method.

10. A method according to any of claims 6 to 9, wherein the normalization of the expression profile is achieved by correcting the expression data for experimental variations with the help of expression data of a control gene or element which is not affected by the tumour state, preferably by calculating the ratio of the expression data of each gene or element in the array of claim 1 or 2 with the expression of a control gene or element or the mean of a pool of control genes or elements.
